# EUROPÄISCHE PATENTSCHRIFT

(11) **EP 1 638 577 B1**
(45) Veröffentlichungstag und Bekanntmachung des Hinweises auf die Patenterteilung: **15.02.2012**
(21) Anmeldenummer: 04739522.3
(22) Anmeldetag: 02.06.2004
(51) Int. Cl.: A61K 31/655, A61K 31/155, A61K 31/4706, A61K 31/352, A61K 31/365, A61P 33/02

(54) **PHARMAZEUTISCHES PRÄPARAT ZUR BEHANDLUNG VON PARASITÄREN TROPENKRANKHEITEN ENTHALTEND DIMINAZEN-DI-ACETURAT**
PHARMACEUTICAL PREPARATION FOR TREATING TROPICAL PARASITIC DISEASES CONTAINING DIAMINAZEN-DIACETURATE
PREPARATION PHARMACEUTIQUE DESTINEE AU TRAITEMENT DE MALADIES TROPICALES PARASITAIRES CONTENANT DIMINAZENE DIACETURATE

(30) Priorität: 03.06.2003 DE 10325672
(43) Veröffentlichungstag der Anmeldung: 29.03.2006
(73) Patentinhaber: Tropmed GmbH, 19370 Parchim (DE)
(72) Erfinder: BOURDICHON, Alain-Jacques, 22303 Hamburg (DE)
(74) Vertreter: Ziebig, Marlene
(86) Internationale Anmeldenummer: PCT/EP2004/005942
(87) Internationale Veröffentlichungsnummer: WO 2004/105772

(56) Entgegenhaltungen:
- WO-A-00/30611
- WO-A-97/06769
- MUKHOPADHYAY RITA ET AL: "Antileishmanial Activity of Berenil and Methylglyoxal bis (Guanylhydrazone) and its Correlation with S-Adenosylmethionine Decarboxylase and Polyamines" INTERNATIONAL JOURNAL OF BIOCHEMISTRY AND CELL BIOLOGY, Bd. 27, Nr. 1, 1995, Seiten 55-59, XP002298912 ISSN: 1357-2725
- WERBOVETZ KARL A: "Promising therapeutic targets for antileishmanial drugs." EXPERT OPINION ON THERAPEUTIC TARGETS. AUG 2002, Bd. 6, Nr. 4, August 2002 (2002-08), Seiten 407-422, XP009037434 ISSN: 1472-8222
- COOMBS G H ET AL: "Leishmania mexicana: drug sensitivities of promastigotes and transforming amastigotes" JOURNAL OF ANTIMICROBIAL CHEMOTHERAPY 1983 UNITED KINGDOM, Bd. 11, Nr. 2, 1983, Seiten 151-162, XP009037480
- YANG D M ET AL: "Effects of qinghaosu (artemisinin) and its derivatives on experimental cutaneous leishmaniasis" PARASITOLOGY, Bd. 106, Nr. 1, 1993, Seiten 7-11, XP009037471 ISSN: 0031-1820

## Beschreibung

Die Erfindung betrifft ein pharmazeutisches Präparat zur Verwendung bei der Behandlung von parasitären Tropenkrankheiten.

In Ländern mit tropischem Klima sind Infektionskrankheiten, die durch Protozoen und Protomonadinen hervorgerufen werden, weit verbreitet, wie zum Beispiel Leishmania, Trypanosomiasis und Malaria. Derartige Erkrankungen führen ohne Behandlung mit hoher Wahrscheinlichkeit zum Tode. Die Behandlung dieser Krankheiten wird chemotherapeutisch durchgeführt.

Während Malaria, Schlafkrankheit und Lepra inzwischen auch dem medizinischen Laien ein Begriff sind, ist die Leishmaniose noch weitgehend unbekannt. Dabei sind rund 350 Millionen Menschen in fast 90 Ländern von dieser Tropenseuche bedroht. Jedes Jahr, so schätzt die Weltgesundheitsorganisation (WHO), treten rund zwei Millionen neue Infektionen auf. Verursacht wird die tödliche Infektion durch einzellige Parasiten, die Leishmanien. Als Wirt fungieren verschiedene Nagetiere, Hunde, Pferde, Schweine, blutsaugende Stechmücken oder Sandfliegen (P.perniciosus, P.ariasi, P.perfiliewi, P.longicuspis, P.neglectus, P.tobbi, P.kandelakii, P.syriacus, P.langeroni), von denen der Erreger auf den Mensch übertragen wird. Die Seuche tritt in vier Formen auf. Besonders gefährlich ist die vizerale Leishmaniose, die als Kala Azar, die Schwarze Krankheit, bekannt ist. Die Parasiten breiten sich dabei rasch in den Organen aus und im Knochenmark bringen sie die Blutbildung quasi zum Erliegen. Verschiedene pharmazeutische Präparate, wie Pentostam®, Glucantime®, Amphotericin B, werden zur Behandlung von Leishmania donovani, Leishmania infantum, Leishmania archibaldi - mit allerdings nur mäßigem Erfolg - verwendet.

Zudem erweisen sich durch die Erreger ausgebildete Resistenzen gegenüber bis dahin gut wirksamen Medikamenten, wie beispielsweise Chloroquine und Artemether, als problematisch, so dass ständig Bedarf an neuen und auch wirksameren Medikamenten besteht.

In der WO 00/30611 wird ein pharmazeutisches Präparat beschrieben, das Diminazen-di-aceturat und Procain enthält und zur Behandlung von Protozoeninfektionen geeignet ist.

Es ist daher Aufgabe der Erfindung, wirksame pharmazeutische Präparate zur Verwendung bei der Behandlung von parasitären Tropenkrankheiten wie Leishmania, Malaria und Typanomiasis zur Verfügung zu stellen, .

Diese Aufgabe wird durch die Merkmale des Ansprüche 1 und 2 gelöst.

Dazu ist erfindungsgemäß ein pharmazeutisches Präparat zur Verwendung bei der Behandlung von parasitären Tropenkrankheiten vorgesehen, das Diminazen-di-aceturat und Chloroquin enthält.

Das Chloroquin wird vorzugsweise in Salzform, beispielsweise als Sulfat oder Phosphat eingesetzt.

Es ist erfindungsgemäß ein weiteres pharmazeutisches Präparat vorgesehen, das Diminazen-di-aceturat und zumindest einen Wirkstoff aus der Gruppe Artemether, Artemisinin, Artheether und Artesunat enthält, wobei Artemether und Artemisinin bevozugt verwendet werden.

Die Präparate sind vorteilhafterweise zur Therapie und auch Prophylaxe verschiedener Tropenkrankheiten geeignet. Dazu zählen u.a Leishmania, Malaria, Trypanosomiasis und Borelliosen, wobei die Präparate schon in geringen Dosierungen äußerst wirksam sind.

Vorzugsweise enthält das jeweilige Präparat zusätzlich Procain oder ein Procainderivat und/oder Lidocain oder ein Lidocainderivat, da durch diese Kombination die Wirksamkeit des Präparates nochmals wesentlich verstärkt wird.

Nach einer bevorzugten Ausführungsform weist das Präparat einen antibiotischen Wirkstoff auf, da bei Protozoeninfektionen stets die Gefahr von Sekundärinfektionen durch andere Erreger, wie Bakterien oder dergleichen aufgrund der naturgemäß stark geschwächten Abwehrkräfte besteht.

Vorzugsweise wird Penicillin oder ein Penicillinderivat verwendet. Vorteilhaft ist hier insbesondere die Verwendung in Form von Procain-Benzylpenicillinat, da dadurch die Wirkdauer des Penicillins verlängert wird.

Auch der Einsatz von Oxytetracyclin oder Tetracyclin ist erfindungsgemäß bevorzugt, da hier zusätzlich noch die Wirksamkeit gegenüber der Anaplosmosis gegeben ist.

Es können aber auch andere antibiotisch wirkende Stoffe, wie zum Beispiel Nisin eingesetzt werden.

Nach einer weiteren bevorzugten Ausführungsform enthält das Präparat weiterhin Phenazon (Antipyrin), das als Analgetikum wirkt, wodurch Schmerz- und Fieberzustände, die durch die Injektion bzw. die Infektion bedingt sind, gelindert werden. Ebenso kann Acetylsalicylsäure bzw. ein entsprechendes Derivat eingesetzt werden.

Personen, die HIV positiv sind, weisen üblicherweise eine erhöhte Anfälligkeit für Sekundärinfektionen, das heißt auch für Protozoeninfektionen auf. Daher kann das Präparat nach einer Ausführungsform zusätzlich eine antiretrovirale Substanz, vorzugsweise Azidothymidin (AZT) aufweisen.

Das pharmazeutisches Präparat kann grundsätzlich in jeder beliebigen und geeigneten Zubereitungsform verabreicht werden. Besonders geeignet und wirksam ist eine liposomale Zubereitung des Präparates, da das Präparat, das mittels Liposomen verabreicht wird, um mehr als 50% wirksamer und die Toxizität um mehr als 50% reduziert ist.

Ansonsten werden vorzugsweise nicht-wässrige oder wässrige Lösungen zur Injektion eingesetzt. Weiterhin ist die Verabreichung in Form von Tabletten, Dragees oder Salbe geeignet. Das pharmazeutische Präparat kann zudem übliche Träger- und Hilfsstoffe enthalten.

Die erfindungsgemäßen Präparate sind gegenüber folgenden Protozoen wirksam:
- Leishmania donovani, L. infantum, L. archibaldi, L. major, L. tropica, L. braziliensis, L. chagassi. L. mexicana,
- P. plasmodium, P. vinckei, P. vivax, P. malariae, P. ovalae,
- Trypanosomes congolense, T. vivax, T. bruccei, T. evansi,
- Piroplasma motasi, P. caballi,
- Babesia bigemina, B. motasi, B. canis und andere Babesien,
- Theileria annulata,
- Borellia recurrens, B. duttoni.

Nachstehend werden bevorzugte Ausführungsformen der Präparate in Form von Injektionslösungen und Tabletten aufgeführt:
1 ml Injektionslösung enthält:

| | |
|---|---|
| Diminazen-di-aceturat | 70 mg |
| Chloroquin Base | 50 mg |
| Procain HCl | 20 mg |
| Antipyrin | 80 mg |

1 Tablette enthält:

| | |
|---|---|
| Diminazen-di-aceturat | 350 mg |
| Chloroquin Base | 250 mg |
| Antipyrin | 400 mg |

1 ml Injektionslösung enthält:

| | |
|---|---|
| Diminazen-di-aceturat | 70 mg |
| Artemether | 40 mg |
| Procain HCl | 20 mg |
| Antipyrin | 80 mg |

1 Tablette enthält:

| | |
|---|---|
| Diminazen-di-aceturat | 350 mg |
| Artemether | 200 mg |
| Antipyrin | 400 mg |

Die zu verabreichende Dosis beträgt vorzugsweise 10 bis 30 ml, vorzugsweise 20 ml / 100 kg bzw. ½ bis 1 ½ Tablette, vorzugsweise 1 Tablette / 100 kg.

Zur Therapie der Leishmania wird weiterhin erfindungsgemäß vorgeschlagen, Diminazen-di-aceturat zur Behandlung der Leishmania zu verwenden, wobei vorzugsweise Procain oder ein Procainderivat und/oder Lidocain oder ein Lidocainderivat in Kombination mit den vorgenannten Wirkstoffen verwendet wird. Dadurch wird die Wirkung von Diminazen-di-aceturat nochmals verstärkt.

Das Procain oder Lidocain bzw. deren Derivate dienen dazu, das mit der Protozoeninfektion auftretende hohe Fieber abzusenken und die gegebenenfalls mit der Injektion verbundenen starken Schmerzen zu vermeiden, die zu tödlichen Schockzuständen führen können. Von wesentlich größerer Bedeutung, wie oben beschrieben, ist jedoch eine verstärkende Wirkung auf den bioziden Wirkstoff Diminazen-di-aceturat..

Dadurch wird eine sichere Abtötung der Protozoen bzw. Leishmanien, insbesondere der Leishmania donovani, L.infantum, L.archibaldi, L.mexicana, L.major, L.tropica, L.braziliensis, L.chagassi gewährleistet, die jedoch auch bei alleiniger Gabe von Diminazen-di-aceturat gegeben wäre, allerdings in höheren Dosierungen.

In vitro Tests haben gezeigt, dass Diminazen-di-aceturat allein oder in Kombination mit Procainhydrochlorid in einer Konzentration von 3,5 µg/ml und 7µg/ml wirksam genug war, um 2 Millionen Leishmania promastogotes per ml nach 7 Tagen vollständig abzutöten. In der nicht behandelten Kontrollgruppe ist innerhalb von 15 Tagen die Parasitenpopulation von 2 Millionen auf 25 Millionen angewachsen.
Das erfindungsgemäße Präparat bzw. die erfindungsgemäße Verwendung finden sowohl im human- wie auch im veterinärmedizinischen Bereich Anwendung.

Weitere vorteilhafte Ausgestaltungen sind in den Unteransprüchen gekennzeichnet.

## Patentansprüche

1. Pharmazeutisches Präparat zur Verwendung bei der Behandlung von parasitären Tropenkrankheiten, **dadurch gekennzeichnet dass** das Präparat Diminazen-di-aceturat und Chloroquin enthält.

2. Pharmazeutisches Präparat zur Verwendung bei der Behandlung von parasitären Tropenkrankheiten, **dadurch gekennzeichnet, dass** das Präparat Diminazen-di-aceturat und zumindest einen Wirkstoff aus der Gruppe Artemether, Artemisinin, Artheether und Artesunat enthält.

3. Pharmazeutisches Präparat nach Anspruch 1 oder 2, **dadurch gekennzeichnet, dass** das Präparat zusätzlich Procain oder ein Procainderivat und/oder Lidocain oder ein Lidocainderivat enthält.

4. Pharmazeutisches Präparat nach einem der Ansprüche 1 bis 3, **dadurch gekennzeichnet, dass** das Präparat zusätzlich einen antibiotischen Wirkstoff enthält.

5. Pharmazeutisches Präparat nach einem der Ansprüche 1 bis 4, **dadurch gekennzeichnet, dass** das Präparat zusätzlich AZT enthält.

6. Pharmazeutisches Präparat nach einem der Ansprüche 1 bis 5, **dadurch gekennzeichnet, dass** das Präparat zusätzlich Phenazon (Antipyrin) und/oder Acetylsalicylsäure bzw. ein entsprechendes Derivat enthält.

## Claims

1. A pharmaceutical preparation for use in the treatment of parasitic tropical diseases, **characterized in that** the preparation contains diminazene di-aceturate and chloroquine.

2. A pharmaceutical preparation for use in the treatment of parasitic tropical diseases, **characterized in that** the preparation contains diminazene di-aceturate and at least one active ingredient from the group of artemether, artemisinin, artheether and artesunate.

3. The pharmaceutical preparation as claimed in claim 1 or 2, **characterized in that** the preparation additionally contains procaine or a procaine derivative and/or lidocaine or a lidocaine derivative.

4. The pharmaceutical preparation as claimed in any of claims 1 to 3, **characterized in that** the preparation additionally contains an antibiotic agent.

5. The pharmaceutical preparation as claimed in any of claims 1 to 4, **characterized in that** the preparation additionally contains AZT.

6. The pharmaceutical preparation as claimed in any of claims 1 to 5, **characterized in that** the preparation additionally contains phenazone (antipyrine) and/or acetylsalicylic acid or a corresponding derivative.

## Revendications

1. Préparation pharmaceutique destinée à être utilisée dans le traitement des maladies tropicales parasitaires, **caractérisée en ce que** la préparation contient du diminazène diacéturate et de la chloroquine.

2. Préparation pharmaceutique destinée à être utilisée dans le traitement des maladies tropicales parasitaires, **caractérisée en ce que** la préparation contient du diminazène diacéturate et au moins un principe actif choisi parmi le groupe comprenant l'artéméther, l'artémisinine, l'artééther et l'artésunate.

3. Préparation pharmaceutique selon la revendication 1 ou 2, **caractérisée en ce que** la préparation contient en plus de la procaïne ou un dérivé de procaïne et/ou de la lidocaïne ou un dérivé de lidocaïne.

4. Préparation pharmaceutique selon l'une quelconque des revendications 1 à 3, **caractérisée en ce que** la préparation contient en plus un principe actif antibiotique.

5. Préparation pharmaceutique selon l'une quelconque des revendications 1 à 4, **caractérisée en ce que** la préparation contient en plus de l'AZT.

6. Préparation pharmaceutique selon l'une quelconque des revendications 1 à 5, **caractérisée en ce que** la préparation contient en plus du phénazone (antipyrine) et/ou de l'acide acétylsalicylique ou un dérivé correspondant.
